# EUROPEAN PATENT SPECIFICATION

(11) **EP 3 452 050 B1**
(45) Date of publication and mention of the grant of the patent: **23.11.2022**
(21) Application number: 17792540.1
(22) Date of filing: 05.05.2017
(51) Int. Cl.: A61K 31/702, A61K 31/7012, A61P 1/12, A23C 9/20, A23L 33/125

(54) **COMPOSITION COMPRISING HMOS FOR THE TREATMENT OF NON-INFECTIOUS DIARRHOEA**
ZUSAMMENSETZUNG MIT HMOS ZUR BEHANDLUNG VON NICHTINFEKTIÖSER DIARRHÖ
COMPOSITION COMPRENANT DU HMOS POUR LE TRAITEMENT DE LA DIARRHÉE NON INFECTIEUSE

(30) Priority: 05.05.2016 US 201615147112
(43) Date of publication of application: 13.03.2019
(73) Proprietor: Glycom A/S, 2970 Hørsholm (DK)
(72) Inventor: MCCONNELL, Bruce, 1814 La Tour de Peilz (CH); VIGSNÆS, Louise Kristine, 2400 Copenhagen NV (DK); ELISON, Emma, 24564 Hjärup (SE)
(74) Representative: Inspicos P/S
(86) International application number: PCT/DK2017/050145
(87) International publication number: WO 2017/190754

(56) References cited:
- EP-A1- 2 842 560
- WO-A1-2012/092155
- WO-A1-2013/032674
- WO-A1-2013/154725
- WO-A1-2015/095747
- WO-A1-2015/157098
- WO-A1-2016/066175
- WO-A1-2017/084673
- US-A1- 2012 171 165
- EMMA ELISON ET AL: "Oral supplementaion of healty adults with 2'- O-fucosyllactose and lacto-N-neotetraose is well tolerated and shifts the intestinal microbiota", BRITISH JOURNAL OF NUTRITION, vol. 116, no. 8, 10 October 2016 (2016-10-10), pages 1356-1368, XP055616188, UK ISSN: 0007-1145, DOI: 10.1017/S0007114516003354
- IRIBARREN CRISTINA ET AL: "1142 - The Effects of Human Milk Oligosaccharides on Bifidobacteria and Gastrointestinal Symptoms in Irritable Bowel Syndrome Patients: A Parallel, Double Blind, Randomized, Placebo-Controlled Trial", GASTROENTEROLOGY : OFFICIAL PUBLICATION OF THE AMERICAN GASTROENTEROLOGICAL ASSOCIATION, vol. 156, no. 6, 2019, XP085677430, ISSN: 0016-5085, DOI: 10.1016/S0016-5085(19)37409-8
- HEITKEMPER M. ET AL.: 'Update on irritable bowel syndrome program of research' JOURNAL OF KOREAN ACADEMY OF NURSING vol. 43, no. 5, 2013, pages 579 - 586, XP055436149
- ROCKOVA S. ET AL.: 'Inter-species differences in the growth of bifidobacteria cultured on human milk oligosaccharides' FOLIA MICROBIOLOGICA vol. 57, no. 4, 2012, pages 321 - 324, XP035080290

## Description

### FIELD OF THE INVENTION

This invention relates generally to compositions and methods for the treatment of non-infectious diarrhoea.

### BACKGROUND TO THE INVENTION

Diarrhoea affects most individuals at some time during their lives. It results when the efficiency of the intestine for absorbing water, electrolytes, and nutrients is impaired. Approximately 8-9 litres of fluid enters the intestines daily of which 1-2 litres represents food and liquid intake, and the rest is from endogenous sources such as salivary, gastric, pancreatic, biliary, and intestinal secretions. Most of the fluid, about 6-7 litres, is absorbed in the small intestine, and only about 1-2 litres is presented to the colon. Most of this is absorbed as it passes through the colon, leaving a stool output of about 100-200 g/day. Therefore, small changes in this absorption efficiency can radically change the wetness of the stool. A great variety of drugs, toxins, pathogens, and foodstuffs can impair the efficiency of electrolyte and water absorption, leading to diarrhoea.

Normal bowel frequency ranges from three times a day to three times a week in the healthy population. Diarrhoea is the increased frequency of stooling, with stool consistency less solid than normal. Acute diarrhoea is defined as three or more stools per day of decreased form from the normal, lasting for less than 14 days. If the duration of symptoms is longer than 1 month, it is considered chronic diarrhoea. Most cases of acute diarrhoea are self-limited, caused by infectious agents (e.g. viruses, bacteria, parasites), and do not require medication unless the patient is immunocompromised.

Mechanistically, absorption may be impaired by poorly absorbed, osmotically active solutes in the intestinal lumen, by alteration in absorptive cell function, by increases in crypt cell secretion, and by too rapid transit of intestinal contents. Most often, absorption is impaired by mechanisms acting in concert. For example, excessive volume of intestinal contents can speed intestinal transit; cytokines from epithelial inflammatory cells can enhance cryptal secretion, and can influence the enteric nervous system to speed transit; bile salts, and long-chain fatty acids, malabsorbed in the small intestine, can block water and electrolyte absorption in the colon. The colon employs several mechanisms to ensure it delivers to the recto sigmoid colon a formed stool, probably the most important factor in faecal continence. The colon has reserve capacity by which it can absorb 2-3 extra litres of water and electrolytes delivered from the small intestine in a day. Colonic bacteria ferment soluble carbohydrate and protein, which escaped small intestinal absorption, into absorbable gases and short-chain fatty acids. Otherwise, these unfermented, unabsorbable solutes would be osmotically active in colonic contents, and would cause diarrhoea.

Most cases of diarrhoea, especially acute diarrhoea, are infection related; especially due to *Campylobacter, Salmonella, Shigella, E. coli, Vibrio* and *Aeromonas* organisms. In most cases, the diarrhoea resolves itself within 48 hours and no treatment other than rehydration is needed. Causes of non-infectious diarrhoea include inflammatory bowel disease, irritable bowel syndrome, ischemic bowel disease, partial small bowel obstruction, pelvic abscess in the recto sigmoid area, faecal impaction, carcinoid syndrome, food allergies, lactose intolerance, the ingestion of poorly absorbable sugars such as lactulose, acute alcohol ingestion, and adverse effects of prescription medication; often antibiotics and chemotherapy/radiation. Non-infectious diarrhoea is often persistent, chronic and difficult to treat. Common treatments include anti-motility agents like loperamide to reduce the number of stools, bile acid sequestrants, digestive enzymes containing lactase, probiotics, etc.

Non-infectious diarrhoea is particularly common in irritable bowel syndrome (IBS) patients. Irritable bowel syndrome is a clinically heterogeneous disorder of human patients, particularly adult, with chronic symptoms such as abdominal pain, abdominal discomfort, abdominal bloating, fatigue, and changes in bowel movement patterns, such as patterns of loose or more frequent bowel movements, diarrhoea and constipation. Routine clinical tests on patients typically show no abnormalities, although their bowels may be more sensitive to certain stimuli, such as balloon insufflation testing. The worldwide prevalence of IBS is about 10-20 % (Longstreth et al. Gastroenterology 130, 1480 (2006)) but may be higher in certain countries. The causes of IBS are unknown but disruptions of the brain-gut axis, acute gastrointestinal infections, small intestinal bacterial overgrowths, antibiotic usages and dysbiosis are thought to be important risk factors (Kim et al. Digest. Dis. Sci. 57, 3213 (2012)). Other risk factors are young age, prolonged fever, anxiety, and depression. Chronic low-grade inflammation commonly occurs in IBS patients, but there are otherwise little or no observable clinical manifestations.

Diagnosis of IBS is difficult. No biomarker-based tests can be performed to diagnose IBS. Diagnosis generally involves excluding conditions that produce IBS-like symptoms and then following a procedure to categorise a patient's symptoms. Ruling out parasitic infections, lactose intolerance, and coeliac disease is recommended for all patients before a diagnosis of IBS is made. Once diagnosed, patients are usually classified in accordance with the Rome IV criteria into four symptom subtypes based on stool consistency: diarrhoea-predominant (IBS-D), constipation-predominant (IBS-C), mixed subtype (IBS-A or IBS-M) with alternating episodes of both diarrhoea and constipation, and unsubtyped IBS (IBS-U).

There is no cure for IBS and current treatments focus on attempting to relieve symptoms. Treatments take various forms such as dietary adjustments, medication, and psychological interventions. Patient education and good doctor-patient relationships are also important. However, most treatment is unsatisfactory and most patients continue to experience chronic pain, fatigue, and other symptoms. While IBS has no direct effect on life expectancy, its high prevalence and significant effects on quality of life make it a condition with a high social cost. The general hopelessness associated with IBS is a source of frustration for both patients and health care practitioners treating them.

Current research has implicated the gastrointestinal microbiota, the brain-gut axis and the mast cells in the pathophysiology of IBS. The human gastrointestinal microbiota includes at least 1000 species of bacteria, and about 10¹⁴ individual bacterial cells from about 160 different species inhabit each individual's intestine (Qin et al. Nature 464, 59 (2010)). It is believed that an individual's genetic make-up and acquired immunity, as well as environmental factors, influence their gastrointestinal microbiota. The microbiota in turn shape the individual's immunity and physiology within the gastrointestinal system. It is also believed that a healthy individual maintains a symbiotic relationship with the microbiota colonizing his/her intestines, while an individual with IBS has an imbalance in this microbiota-host interaction.

Treatments that target gastrointestinal microbiota such as antibiotics, probiotics and prebiotics appear to alleviate the symptoms of IBS; at least temporarily. For instance, the antibiotic rifaximin appears to reduce bowel movement in IBS-D patients.

Abdominal pain and discomfort associated with IBS is connected to the brain-gut axis and the response to stress hormones. IBS patients typically experience abnormal gut motility and visceral hypersensitivity mediated by the brain-gut axis or central stress response system. One arm of the brain-gut axis is the central efferent pathway, which is formed by the sympathetic nervous system and the hypothalamic-pituitary-adrenal axis (HPA). In stress-sensitive disorders including IBS, stress hormones of the HPA axis, such as adrenocorticotropic hormone (ACTH), cortisol, and catecholamine are released. Some studies have shown that the HPA axis response in IBS patients is caused by increased mucosal immune activation, which in turn increases plasma cytokine levels to stimulate the HPA axis.

In addition to the gut microbiome and the gut-brain axis, the mast cells may also play an important role in the pathogenesis of IBS. Increased mast cell infiltration and activation in distal gut segments are associated with symptom onset and severity of IBS. These cells are also implicated in the elevated response of visceral afferent nerves to mucosal stimulus in IBS patients. Mast cell hyperplasia is commonly observed following infection by bacteria in both post-infectious IBS and non-post-infectious IBS.

A recent development in IBS treatment has been the low FODMAP diet. This diet requires patients to restrict the intake of FODMAP carbohydrates. These are Fermentable Oligo-, Di-, Monosaccharides and Polyols which are poorly absorbed in the proximal small intestine, osmotically active, and fermented by intestinal bacteria in which some produce hydrogen. Adherence to this diet has resulted in symptom improvements for some patients (Staudacher et al. J. Hum. Nutr. Diet. 24, 487 (2011)). However, some of the FODMAP carbohydrates are beneficial fibres, and foods that contain them are common, highly nutritious fruits, vegetables and legumes.

Similarly, for lactose intolerance, there is no universally accepted therapy for treatment. However, existing strategies for management of the conditions include avoidance of lactose-containing dairy foods (milks, soft cheeses and ice creams) and consuming lactase prior to a meal containing lactose. Although restricting dietary lactose may improve gastrointestinal complaints, long-term effects of a diet low or free of dairy products can be of concern, since dairy products provides a package of essential nutrients such as calcium, protein, riboflavin, vitamin A and vitamin D. Dietary calcium is an important part of the recommended daily allowance of vitamins and minerals, and for many people it is not possible to achieve recommended daily calcium intakes with a dairy low or free diet. It has been observed that deficiency in calcium can lead to increased risk of osteoporosis, hypertension and possibly cancer.

Lactase drugs are effective and are available without prescription. However, they do need to be consumed before meals containing lactose. Also, although uncommon, they may provoke serious, allergy-related side effects.

Non-infectious diarrhoea caused by chemotherapy or radiation is a common problem in cancer patients. The incidence of chemotherapy-induced diarrhoea (CID) has been reported to be as high as 50-80 % of treated cancer patients. CID can cause depletion of fluids and electrolytes, malnutrition, dehydration and hospitalization, all of which can lead to cardiovascular problems and death. CID is caused by acute damage to the intestinal mucosa (including loss of intestinal epithelium, superficial necrosis and inflammation of the bowel wall) which causes an imbalance between absorption and secretion in the small bowel leading to excessive electrolyte and fluid secretion. Prevention of CID is very limited and only a few treatment are recommended in current guidelines: loperamide, deodorized tincture of opium and octreotide. However, these treatments only targets symptoms and can be used only temporarily (Stein et al. Ther. Adv. Med. Oncol. 2, 51 (2010)).

WO 2015/095747 discloses the use of nutritional compositions comprising inter alia selected human milk oligosaccharide to prevent or treat dehydration.

Elison et al. Br. J. Nutr. 116, 1356 (2016) showed that 2'-FL and LNnT are safe and well tolerated in healthy adults, specific modulators of the adult microbiota, and may be a valuable tool to restore homoeostasis in adults having an imbalanced microbiota.

WO 2017/084673 relates to a synthetic composition comprising human milk oligosaccharides for use in at least partially restoring the commensal gastrointestinal microbiota and preventing or mitigating antibiotic associated diarrhoea.

Therefore, there is a need for a safe, effective intervention for the treatment of non-infectious diarrhoea.

### SUMMARY OF THE INVENTION

This invention provides synthetic compositions comprising one or more human milk oligosaccharides (HMOs), that can be advantageously used for prophylaxis or treatment of non-infectious diarrhoea in a human, in particular a non-infant human individual, wherein the human is a lactose intolerant patient.

Accordingly:
- a first aspect of this invention relates to a human milk oligosaccharide or a mixture of human milk oligosaccharides for the prophylaxis or treatment of non-infectious diarrhoea in a human, wherein the human is a lactose intolerant patient; and
- a second aspect of this invention relates to a synthetic composition for the prophylaxis or treatment of non-infectious diarrhoea in a human, wherein the human is a lactose intolerant patient, the composition comprising an effective amount of one or more human milk oligosaccharides.

Preferably, the amount of a human milk oligosaccharide is effective to increase (i) the abundance, particularly the relative abundance, of bifidobacteria, and/or (ii) the beta-galactosidase activity, in the gastrointestinal tract of the human. More preferably, in a period of about 14 days of treatment the bifidobacteria increased is a member of the phylogenetic *Bifidobacterium adolescentis* group, for example, *Bifidobacterium pseudocatenulatum* and/or *Bifidobacterium adolescentis,* and, after about 14 days of treatment, are *Bifidobacterium longum* and/or *Bifidobacterium bifidum.*

The patient may have intestinal dysbiosis and/or an impaired mucosal barrier.

Preferably, the human milk oligosaccharide is 2'-FL, 3-FL, DFL, LNnT, LNT, 3'-SL, 6'-SL or LNFP-I or a mixture thereof. For example, the composition can comprise a mixture of a fucosylated HMOs such as 2'-FL and/or DFL, and a non-fucosylated neutral HMO such as LNnT or LNT, or both. In one preferred embodiment, the human milk oligosaccharide is a mixture of 2'-FL and LNnT and/or LNT. In this embodiment, the 2'-FL and LNnT/LNT may be present in a mass ratio of about 5:1 to 1:1; more preferably about 4:1 to 2:1. In another preferred embodiment, the human milk oligosaccharide is a mixture of 2'-FL and/or DFL and LNnT and/or LNT. In this embodiment, the 2'-FL/DFL and LNnT/LNT may be present in a mass ratio of about 5:1 to 1:1; more preferably about 4:1 to 2:1.

Preferably, the one or more HMOs are administered to a human in need in two steps:
(a) in a first step, during an initial treatment period of about 14 days, to increase the relative abundance of bifidobacteria of the phylogenetic *Bifidobacterium adolescentis* group; and
(b) in a second step, during an additional period of treatment of 1 or more days following the initial treatment period , to increase the relative abundance of *Bifidobacterium longum* and/or *Bifidobacterium bifidum,*
in the microbiota in the gastro-intestinal tract of said human.

The synthetic composition can be a nutritional or pharmaceutical composition. Preferably, synthetic composition of the invention is administered daily. Furthermore, the synthetic composition is preferably administered for a period of at least one month, such as at least 2 months or for a longer period of time, for example chronically on an ongoing basis.

The synthetic composition may be administered to the human or patient as a daily dose of about 1 g to about 15 g such as from about 3 g to about 10 g of HMOs. The patient can receive a higher amount, preferably 5 g to 10 g per day, of the HMOs for up to 8 weeks, followed by a lower amount, preferably 1 g to 5 g per day, for a period of time afterwards, if necessary, which period may be from 1 week to 1 month or even longer.

### DETAILED DESCRIPTION OF THE INVENTION

It has been surprisingly found that human milk oligosaccharides (HMOs) are able to decrease bowel movement frequency and improve stool consistency in patients suffering from non-infectious diarrhoea, particularly those who are suffering from intestinal dysbiosis or an impaired mucosal barrier. HMOs preferentially increase the abundance of bifidobacteria in the gastro-intestinal tract, metabolising carbohydrates, in particular, which escaped absorption in the small intestine, into lactate and short-chain fatty acids, making them less osmotically active in the colon. Further, HMOs act to reduce chronic mucosal inflammation and/or repair damage to the mucosal barrier, potentially reducing cryptal secretion. HMOs can also act on neuronally dependent gut migrating motor complexes to address disorders of gut motility and possibly have beneficial effects on the central nervous systems of patients. As an outcome, bowel movement frequency is decreased and stool consistency improved.

The term "oral administration" preferably means any conventional form for the oral delivery of a composition to a patient that causes the deposition of the composition in the gastrointestinal tract (including the stomach) of the patient. Accordingly, oral administration includes swallowing of composition by the patient, enteral feeding through a naso-gastric tube, and the like.

The term "effective amount" preferably means an amount of a composition that provides a human milk oligosaccharide in a sufficient amount to render a desired treatment outcome in a patient. An effective amount can be administered in one or more doses to the patient to achieve the desired treatment outcome.

The term "human milk oligosaccharide" or "HMO" preferably means a complex carbohydrate found in human breast milk that can be in acidic or neutral form. More than about 200 different HMO structures are known to exist in human breast milk (Urashima et al.: Milk Oligosaccharides, Nova Biomedical Books, New York, 2011). HMOs can be backbone, fucosylated and sialylated oligosaccharides. Backbone HMOs consists of Glu, Gal and GlcNAc and are devoid of Fuc and sialic acid. Examples of backbone HMOs include lacto-N-tetraose (LNT), lacto-N-neotetraose (LNnT), lacto-N-neohexaose (LNnH) and lacto-N-hexaose (LNH). Fucosyl HMOs are fucosylated lactoses or fucosylated backbone HMOs such as 2'-fucosyllactose (2'-FL), lacto-N-fucopentaose I (LNFP-I), lacto-N-difucohexaose I (LNDFH-I), 3-fucosyllactose (3-FL), difucosyllactose (DFL), lacto-N-fucopentaose III (LNFP-III), fucosyl-para-lacto-N-neohexaose (F-pLNnH), lacto-N-difucohexaose I (LNDFH-I), fucosyl-lacto-N-hexaose II (FLNH-II), lacto-N-fucopentaose V (LNFP-V), lacto-N-difucohexaose II (LNDFH-II), fucosyl-lacto-N-hexaose I (FLNH-I), fucosyl-lacto-N-hexaose III (FLNH-III) and fucosyl-para-lacto-N-neohexaose (F-pLNnH). Sialyl HMOs are sialylated lactoses or sialylated backbone HMOs such as 3',6-disialyllacto-N-tetraose (DSLNT), 6'-sialyllactose (6'-SL), 3'- sialyllactose (3'-SL), 6'-sialyllacto-N-neotetraose (LST c), 3'-sialyllacto-N-tetraose (LST a) and 6-sialyllacto-N-tetraose (LST b). HMOs containing both sialyl and fucosyl groups may be considered to belong to either of the latter two groups. Examples for sialyl and fucosyl HMOs include disialyl- fucosyl-lacto-N-hexaose II (DSFLNH-II), fucosyl-sialyl-lacto-N-neohexaose I (FSLNnH-I), fucosyl-sialyl-lacto-N-hexaose I (FSLNH-I) and 3-fucosyl-3'-sialyllactose (FSL). In context of the present invention, lactose is not included in the group of HMOs.

The terms "microbiota", "microflora" and "microbiome" preferably mean a community of living microorganisms that typically inhabits a bodily organ or part. The most dominant members of the gastrointestinal microbiota include microorganisms of the phyla of *Firmicutes, Bacteroidetes, Actinobacteria, Proteobacteria, Synergistetes, Verrucomicrobia, Fusobacteria,* and *Euryarchaeota;* at genus level the microorganisms of *Bacteroides, Faecalibacterium, Bifidobacterium, Roseburia, Alistipes, Collinsella, Blautia, Coprococcus, Ruminococcus, Eubacterium* and *Dorea;* and at species level microorganisms of *Bacteroides uniformis, Alistipes putredinis, Parabacteroides merdoe, Ruminococcus bromii, Doreo longicatena, Bacteroides caccae, Bacteroides thetaiotaomicron, Eubacterium hollii, Ruminococcus torques, Faecalibacterium prausnitzii, Ruminococcus lactaris, Collinsella aerofaciens, Dorea formicigenerans, Bacteroides vulgatus* and *Roseburia intestinalis.* In some instances, the gastrointestinal microbiota includes the mucosa-associated microbiota, which is located in or attached to the mucus layer covering the epithelium of the gastrointestinal tract, and luminal-associated microbiota, which is found in the lumen of the gastrointestinal tract.

The term "patient" means a human individual of 3 years of age and older that has a non-infectious diarrhoea and is lactose intolerant.

Preferably, the patient has intestinal dysbiosis, such as an impaired microbiota and/or an impaired mucosal barrier.

The term "prophylaxis" means a treatment given or an action taken to prevent a disease in the patient, which disease is associated with or triggered by non-infectious diarrhoea.

The terms "irritable bowel syndrome" and "IBS" preferably mean a group of functional bowel disorders of humans, particularly adults, characterized by one or more chronic symptoms including abdominal pain, abdominal discomfort, abdominal bloating, fatigue, and changes in bowel movement patterns, such as patterns of loose or more frequent bowel movements, diarrhoea and constipation, typically in the absence of any apparent structural abnormality. There are at least three forms of IBS, depending on which symptom predominates: (1) diarrhoea-predominant (IBS-D); (2) constipation-predominant (IBS-C); and (3) IBS with alternating stool pattern (IBS-A or IBS-M). There are also various clinical subtypes of IBS, such as post-infectious IBS (IBS-PI) or unsubtyped IBS (IBS-U). Preferably, the invention relates to IBS-D or/and IBS-A patients.

The term "bifidobacteria" means a member of the *Bifidobacterium* genus commonly found in the human gastro-intestinal tract. Examples of bifidobacteria are *Bifidobacterium longum, Bifidobacterium bifidum,* and members of the phylogenetic *Bifidobacterium adolescentis* group. Bifidobacteria of the phylogenetic *Bifidobacterium adolescentis* group are, for example, *Bifidobacterium pseudocatenulatum* and/or *Bifidobacterium adolescentis.*

The term "beta-galactosidase activity" means enzymatic activity of an enzyme having glycoside hydrolase activity, in particular, lactase, which is involved in the hydrolysis of the disaccharide lactose into constituent galactose and glucose monomers in the human intestine. The beta-galactosidase activity can be measured by analyzing the concentration of released glucose after 30 min reaction in vitro using lactose as substrate. Analytical methods for measuring lactase activity in vitro are well known in the art. Preferably, beta-galactosidase activity measured in the patient samples after administration of a synthetic composition of the invention is increased to at least 15-20 %, more preferably 20-50 %, compared to the enzyme activity measured in samples taken from the patient before the beginning of the administration of one or more HMOs or the synthetic composition comprising one or more HMOs. The term "synthetic composition" means a composition which is artificially prepared and preferably means a composition containing at least one compound that is produced *ex vivo* chemically and/or biologically, e.g. by means of chemical reaction, enzymatic reaction or recombinantly. In some embodiments a synthetic composition may be, but preferably is not, identical with a naturally occurring composition. The synthetic composition typically comprises one or more HMOs that are capable of preferentially increasing the abundance of bifidobacteria. In some embodiments, the synthetic composition may comprise one or more compounds or components other than HMOs that may have an effect on bifidobacteria of a human subject microbiota *in vivo,* e.g. non-digestible oligosaccharides or prebiotics. Also in some embodiments, the synthetic compositions may comprise one or more nutritionally or pharmaceutically active components which do not affect adversely the efficacy of the above mentioned compounds. Some non-limiting embodiments of a synthetic composition of the invention are also described below.

The term "relative abundance of bifidobacteria" means the abundance of bifidobacteria relative to other genus in the microbiota of the gastro-intestinal tract.

The term "relative growth of bifidobacteria" means the growth of bifidobacteria relative to other genus in the microbiota in the gastro-intestinal tract.

The term "non-infant human" or "non-infant" means in the present context a human of 3 years of age and older. A non-infant human can be a child, a teenager, an adult or an elderly.

The term "enteral administration" means any conventional form for delivery of a composition to a human that causes the deposition of the composition in the gastrointestinal tract (including the stomach). Methods of enteral administration include feeding through a naso-gastric tube or jejunum tube, oral, sublingual and rectal.

The term "oral administration" means any conventional form for the delivery of a composition to a human through the mouth. Accordingly, oral administration is a form of enteral administration.

The term "initial period" of treatment with an HMO or an HMO mixture is about 14 days from the start of the treatment; "additional period" of treatment means 1 or more days following the initial period of treatment.

The term "about" in the present context means up to 2.5 % deviation from the corresponded value.

The term "preferably" is used herein to indicate the best mode of invention, but to limit the scope of invention.

### HMOs for prophylaxis or treatment of non-infectious diarrhoea in a human

HMOs for prophylaxis or treatment of non-infectious diarrhoea in a human may be a single HMO, or a mixture of any HMOs suitable for the purpose of the invention. Preferably, the HMO is a fucosylated or a non-fucosylated neutral HMO. More preferably, the HMOs for prophylaxis or treatment of non-infectious diarrhoea in a human is a mixture of at least a first HMO and at least a second HMO, where the first HMO is a fucosylated neutral HMO and the second HMO is a non-fucosylated neutral HMO. Particularly, the mixture contains a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the mixture contains a fucosylated HMO selected from the list consisting of 2'-FL, 3-FL and DFL, and a non-fucosylated HMO selected from the list consisting of LNT and LNnT; advantageously the mixture comprises 2'-FL and/or DFL and LNnT and/or LNT. In some embodiments, the mixture essentially consists of two neutral HMOs, e.g. a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the mixture essentially consists of a fucosylated HMO selected from the list consisting of 2'-FL, 3-FL and DFL, and a non-fucosylated HMO selected from the list consisting of LNT and LNnT; in one preferred embodiment the mixture essentially consists of 2'-FL and LNnT, in another preferred embodiment the mixture essentially consists of 2'-FL and LNT.

The HMOs can be isolated or enriched by well-known processes from milk(s) secreted by mammals including, but not limited to human, bovine, ovine, porcine, or caprine species. The HMOs can also be produced by well-known processes using microbial fermentation, enzymatic processes, chemical synthesis, or combinations of these technologies. As examples, using chemistry LNnT can be made as described in WO 2011/100980 and WO 2013/044928, LNT can be synthesized as described in WO 2012/155916 and WO 2013/044928, a mixture of LNT and LNnT can be made as described in WO 2013/091660, 2'-FL can be made as described in WO 2010/115934 and WO 2010/115935, 3-FL can be made as described in WO 2013/139344, 6'-SL and salts thereof can be made as described in WO 2010/100979, sialylated oligosaccharides can be made as described in WO 2012/113404 and mixtures of human milk oligosaccharides can be made as described in WO 2012/113405. As examples of enzymatic production, sialylated oligosaccharides can be made as described in WO 2012/007588, fucosylated oligosaccharides can be made as described in WO 2012/127410, and advantageously diversified blends of human milk oligosaccharides can be made as described in WO 2012/156897 and WO 2012/156898. With regard to biotechnological methods, WO 01/04341 and WO 2007/101862 describe how to make core human milk oligosaccharides optionally substituted by fucose or sialic acid using genetically modified *E. coli.*

### Synthetic composition comprising HMOs

The synthetic composition may comprise a single HMO, or a mixture of any HMOs suitable for the purpose of the invention. Preferably, the HMO is a fucosylated or a non-fucosylated neutral HMO. More preferably, the composition comprises a mixture of at least a first HMO and at least a second HMO, where the first HMO is a fucosylated neutral HMO and the second HMO is a non-fucosylated neutral HMO. Particularly, the composition may contain a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the composition contains a fucosylated HMO selected from the list consisting of 2'-FL, 3-FL and DFL, and a non-fucosylated HMO selected from the list consisting of LNT and LNnT, advantageously the composition comprises 2'-FL and/or DFL and LNnT and/or LNT. In some embodiments, the composition comprises a mixture essentially consisting of two neutral HMOs, e.g. a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the composition comprises a mixture consisting of a fucosylated HMO selected from the list consisting of 2'-FL, 3-FL and DFL, and a non-fucosylated HMO selected from the list consisting of LNT and LNnT; in one preferred embodiment the composition comprises a mixture essentially consisting of 2'-FL and LNnT, in another preferred embodiment the composition comprises a mixture essentially consisting of 2'-FL and LNT.

A synthetic composition of this invention comprising one or more human milk oligosaccharides can take any suitable form. For example, the composition can be in the form of a nutritional composition which contains other macronutrients such as proteins, lipids or other carbohydrates, preferably where the other carbohydrate is lactose and/or a carbohydrate different than an HMO. The synthetic composition can also be a pharmaceutical composition.

### Nutritional compositions

A nutritional composition of this invention can contain sources of protein, lipids and/or digestible carbohydrates and can be in powdered or liquid forms. The composition can be designed to be the sole source of nutrition or a nutritional supplement. For IBS patients, a nutritional supplement is preferred; especially a supplement which can form a meal or snack replacement. Preferably the nutritional composition is lactose-reduced or, better yet, lactose-free. Preferably, the nutritional composition is also free from, or low in amounts of, FODMAP carbohydrates.

Suitable protein sources include milk proteins, soy protein, rice protein, pea protein and oat protein, or mixtures thereof. Milk proteins can be in the form of milk protein concentrates, whey protein or casein, or mixtures of both. Soy, rice, pea and oat protein can be in the form or protein isolated. The protein can be whole protein or hydrolysed protein, either partially hydrolysed or extensively hydrolysed. The protein can provide about 5 % to about 50 %, preferably about 10 % to 30 %, of the energy of the nutritional composition. The protein source preferably is not a source of non-fermentable carbohydrates such as lactose. Therefore, if a milk protein is used as the protein source, the milk protein is preferably lactose-reduced or lactose-free.

The protein source can be a source of glutamine, threonine, cysteine, serine, proline, or a combination of these amino acids. The glutamine source can be a glutamine dipeptide and/or a glutamine enriched protein. Glutamine can be included due to the use of glutamine by enterocytes as an energy source. Threonine, serine and proline are important amino acids for the production of mucin. Mucin coats the GI tract and can reduce permeability. Cysteine is a major precursor of glutathione, which is key for the antioxidant defences of the body.

Suitable digestible carbohydrates include maltodextrin, hydrolysed or modified starch or corn starch, glucose polymers, corn syrup, corn syrup solids, tapioca, sucrose, and glucose, or mixtures thereof. Generally digestible carbohydrates provide about 35 % to about 75 %, preferably about 45 % to 70 %, of the energy of the nutritional composition. Preferably the digestible carbohydrate is free from lactose.

Suitable lipids include rapeseed oil, sunflower seed oil, palm oil, soy oil, milk fat, corn oil and soy lecithin. Long-chain poly unsaturated fatty acids (LC-PUFA), especially omega-3 fatty acids such as docosahexaenoic acid (DHA), can be included in the lipid source because they have anti-inflammatory properties. Suitable sources of LC-PUFA are plant oils, marine plankton oils, fungal oils, and fish oils. The lipid source can also include medium chain triglycerides (MCT). Fractionated coconut oils are a suitable source of medium chain triglycerides. The lipid source preferably provides about 5 % to about 25 % of the energy of the nutritional composition; for example, about 10 % to 20 %. The lipid content is preferably reduced because high fat diets can provoke IBS symptoms.

The nutritional composition may also include vitamins and minerals. If the nutritional composition is intended to be a sole source of nutrition, it preferably includes a vitamin and mineral profile, preferably a complete vitamin and mineral profile. The term "complete" in the present context means a vitamin and mineral profile comprising all vitamins and minerals essential for body function, wherein the essential vitamins includes at least 9 vitamins from the exemplary group below, such as 10, 11, 12 or 13, or more, and the essential minerals includes at least 5 minerals from the exemplary group below, such as from 6 to 13 or more. Examples of vitamins include vitamins A, B- complex (such as B1, B2, B6 and B12), C, D, E and K, niacin and acid vitamins such as pantothenic acid and folic acid and biotin. Examples of minerals include calcium, iron, zinc, magnesium, iodine, copper, phosphorus, manganese, potassium, chromium, molybdenum, selenium, nickel, tin, silicon, vanadium and boron.

The nutritional composition can also include a carotenoid such as lutein, lycopene, zeaxanthin, and beta-carotene. The total amount of carotenoid included can vary from about 0.001 µg/ml to about 10 µg/ml. Lutein can be included in an amount of from about 0.001 µg/ml to about 10 µg/ml, preferably from about 0.044 µg/ml to about 5 g/ml of lutein. Lycopene can be included in an amount from about 0.001 µg/ml to about 10 µg/ml, preferably about 0.0185 mg/ml to about 5 g/ml of lycopene. Beta-carotene can comprise from about 0.001 µg/ml to about 10 mg/ml, for example about 0.034 µg/ml to about 5 µg/ml of beta-carotene.

The nutritional composition can also contain various other conventional ingredients such as preservatives, emulsifying agents, thickening agents, buffers, fibres and probiotics, especially probiotics which can help to reduce symptoms in IBS patients (e.g. VSL#3, *B. infantis* 35624, *B. animalis* subsp. *lactis* BB-12, *B. lactis* Bi-07, *L. rhamnosus* GG, *L. rhamnosus* Lc705, *L. plantarum* DSM 9843, *L. plantarum* CECT7484, *L. plantarum* CECT7485, *L. acidophilus* NCFM, *L. fermentum* CECT5716, *B. breve* Bb99, *Propionibacterium freundenreichii* ssp. *Shermanii* JS, *P. acidilactici* CECET7483, *Streptococcus faecium*)*,* antioxidant/anti-inflammatory compounds including tocopherols, carotenoids, ascorbate/vitamin C, ascorbyl palmitate, polyphenols, glutathione, and superoxide dismutase (melon), other bioactive factors (e.g. growth hormones, cytokines, TFG-β), colorants, flavours, and stabilisers, lubricants, and so forth.

The nutritional composition can be in the form of a soluble powder, a liquid concentrate, or a ready-to-use formulation. Various flavours, fibres and other additives can also be present.

The nutritional compositions can be prepared by any commonly used manufacturing techniques for preparing nutritional compositions in solid or liquid form. For example, the composition can be prepared from various feed solutions. A protein-in-fat feed solution can be prepared by heating and mixing the lipid source and then adding an emulsifier (e.g., lecithin), fat soluble vitamins, and at least a portion of the protein source while heating and stirring. A carbohydrate feed solution is also prepared by adding minerals, trace and ultra trace minerals, thickening or suspending agents to water while heating and stirring. The resulting solution is held for 10 minutes with continued heat and agitation before adding carbohydrates (e.g. the HMOs and digestible carbohydrate sources). The resulting feed solutions are then blended together while heating and agitating and the pH adjusted to 6.6-7.0, after which the composition is subjected to high-temperature short-time processing during which the composition is heat treated, emulsified and homogenized, and then allowed to cool. Water soluble vitamins and ascorbic acid are added, the pH is adjusted to the desired range if necessary, flavours are added, and water is added to achieve the desired total solid level.

For a liquid product, the resulting solution can then be aseptically packaged to form an aseptically packaged nutritional composition. In this form, the nutritional composition can be in ready-to-feed or concentrated liquid form. Alternatively, the composition can be spray dried and processed and packaged as a reconstitutable powder.

When the nutritional product is a ready-to-feed nutritional liquid, the total concentration of HMOs in the liquid, by weight of the liquid, is from about 0.02 % to about 2.0 %, including from about 0. 1 % to about 1.5 %, including from about 0.3 % to about 1.0 %. When the nutritional product is a concentrated nutritional liquid, the total concentration of HMOs in the liquid, by weight of the liquid, is from about 0.04 % to about 4.0 %, including from about 0.2 % to about 3.0 %, including from about 0.6 % to about 2.0 %.

### Unit dosage forms

The synthetic composition can also be in a unit dosage form such as a capsule, tablet or sachet. For example, the composition can be in a tablet form comprising the human milk mono-and/or oligosaccharides, and one or more additional components to aid formulation and administration, such as diluents, excipients, antioxidants, lubricants, colorants, binders, disintegrants, and the like.

Suitable diluents, excipients, lubricants, colorants, binders, and disintegrants include polyethylene, polyvinyl chloride, ethyl cellulose, acrylate polymers and their copolymers, hydroxyethyl-cellulose, hydroxypropylmethyl-cellulose (HPMC), sodium carboxymethylcellulose, polyhydroxyethyl methacrylate (PHEMA), polyvinyl alcohol (PVA), polyvinyl pyrrolidone (PVP), polyethylene oxide (PEO), or polyacrylamide (PA), carrageenan, sodium alginate, polycarbophil, polyacrylic acid, tragacanth, methyl cellulose, pectin, natural gums, xanthan gum, guar gum, karaya gum, hypromellose, magnesium stearate, microcrystalline cellulose, and colloidal silicon dioxide. Suitable antioxidants are vitamin A, carotenoids, vitamin C, vitamin E, selenium, flavonoids, polyphenols, lycopene, lutein, lignan, coenzyme Q10 ("CoQIO") and glutathione.

The unit dosage forms, especially those in sachet form, can also include various nutrients including macronutrients.

The unit dosage forms can be administered orally, e.g. as a tablet, capsule, or pellet containing a predetermined amount, or as a powder or granules containing a predetermined concentration or a gel, paste, solution, suspension, emulsion, syrup, bolus, electuary, or slurry, in an aqueous or non-aqueous liquid, containing a predetermined concentration. Orally administered compositions can include binders, lubricants, inert diluents, flavouring agents, and humectants. Orally administered compositions such as tablets can optionally be coated and can be formulated so as to provide sustained, delayed or controlled release of the mixture therein.

The unit dosage forms can also be administered by rectal suppository, aerosol tube, naso-gastric tube or direct infusion into the GI tract or stomach.

The unit dosage forms can also include therapeutic agents such as antiviral agents, antibiotics, probiotics, analgesics, and anti-inflammatory agents. The synthetic composition in unit dosage form may be a pharmaceutical composition or a nutritional supplement.

### Administration dosing

For reducing or preventing symptoms of non-infectious diarrhoea in a patient, the amount of HMO(s) required to be administered to the patient will vary depending upon factors such as the risk and severity of the disease, the age of the patient, the form of the composition, and other medications being administered to the patient. However, the required amount can be readily set by a medical practitioner and would generally be in the range from about 200 mg to about 20 g per day, in certain embodiments from about 1 g to about 15 g per day, from about 3 g to about 10 g per day, in certain embodiments from about 3 g to about 7.5 g per day. An appropriate dose can be determined based on several factors, including, for example, body weight and/or condition, the severity of the condition, being treated or prevented, other ailments and/or diseases, the incidence and/or severity of side effects and the manner of administration. Appropriate dose ranges may be determined by methods known to those skilled in the art. During an initial treatment phase, the dosing can be higher or lower depending upon the need to boost bifidobacteria abundance or initial tolerance to HMOs. During a maintenance phase, the dosing can be set for chronic long term use.

The duration of the HMO administration will vary depending upon factors such as the risk and severity of the medical condition, age, the form of the composition, the dose and other medications being administered. However, the duration can be readily set by a medical practitioner. Generally, a duration of at least a week will be required to sufficiently to impact symptoms. For example, the duration may be for 1 to 3 months, or longer. The administration can continue chronically for an indefinite period.

### EXAMPLES

Examples below are to illustrate non-limiting embodiments of the invention.

### Example 1 - Human trial in lactose intolerant patients

A total of 60 male and female with lactose intolerance are recruited to participate in a randomized double-blind, parallel, placebo-controlled study. After a screening visit and run-in period of 1-2 weeks eligible subjects are randomized into two groups, each of 30 participants. One group is administered a placebo product containing 5 grams of glucose, the other group is administered a treatment product containing 5 grams of a 4:1 mass ratio mix of 2'-FL and LNnT for 30 days.

Inclusion criteria includes adults in the age of 18 to 60 years with current or recent self-reported history of dairy intolerance of at least 1-month duration, and with gastrointestinal symptoms after dairy consumption. Exclusion criteria includes participation in a clinical study one month prior to the screening visit; abnormal results in the screening tests which are clinically relevant for study participation; suffering from a severe disease such as malignancy, diabetes, severe coronary disease, kidney disease, neurological disease, or severe psychiatric disease or any condition which can confound the results of the study; ingested anti-, pre- or probiotics 3 month prior to the study; consumed on a regular basis any medication that might interfere with symptom evaluation 2 weeks prior to the study; and pregnant or lactating.

At the screening visit (first visit), medical history and concomitant medication is registered and blood samples for safety analyses are collected. Lactose intolerance is confirmed by a 25-gram lactose challenge, where gastrointestinal symptoms (associated with lactose intolerance) and hydrogen production are assessed via hydrogen breath test (HBT) for 6 hours post-lactose challenge. A positive HBT is defined as a hydrogen gas elevation of 20 parts per million (ppm) at 4 time-points within 6 hours following a lactose-loading dose. A faecal sample kit is distributed, and participants are instructed to keep their collected faecal samples in the freezer until the next visit.

At the second visit, eligibility criteria are checked and eligible subjects are randomised to the two arms in the trial (treatment and placebo group). Faecal samples are collected and stored at -80° C until analysis. Equipment for new faecal samples are distributed. Participants are familiarised with an interactive internet enabled system which records data daily and they are provided with either treatment or placebo products. Subjects are asked not to change their usual diet and to avoid dairy products during the 30-day treatment period.

During the 30-day treatment period the participants consume either a placebo or a treatment product daily. Participants are instructed to consume the products in the morning with breakfast. Compliance is monitored through the interactive internet enabled system. The participants also use the system to record:
- Bristol Stool Form Scale (BSFS) information,
- Gastrointestinal Symptom Rating Scale (GSRS) information.

The GSRS questionnaire includes 15 items covering five dimensions (abdominal pain, indigestion, reflux, diarrhoea, constipation) and uses a seven-graded Likert scale.

At the third visit, after the 30-day treatment period, faecal samples are collected. Additionally, the participants are challenged with 25-gram lactose where lactose digestion are measured by hydrogen production in the HBT and evaluation of gastrointestinal symptoms (associated with lactose intolerance) is measured by participant's self-assessment of symptoms over the 6 hours following the lactose challenge.

After completion of the treatment period, participants are followed for an additional 30 days and instructed to reintroduce dairy foods into their diets. During the follow-up period, participants record BSFS and GSRS information.

At the end of the study, each participant has an exit visit with the medical team and faecal samples are collected.

Lactose digestion is measured by breath hydrogen (BH) production. BH is measured in parts per million (ppm) using a validated hand-held hydrogen chemical sensor (EC60 gastrolyzer, Bedfont Scientific Ltd, United Kingdom). Following a baseline measurement (0 hour), participants ingest 25 g of lactose mixed in water. BH is then remeasured at 30 and 60 min, and at 3 hours and 6 hours after lactose challenge. The baseline value is subtracted from readings recorded at each subsequent time interval. In general, an acceptable baseline value is 20 ppm or lower. A definite positive value is defined as more than 20 ppm above baseline at any time point. Results of each BH measurement are summed to obtain a value for total BH.

Symptoms of lactose intolerance are recorded on a four-point Likert scale at baseline (0 h) and at 3 and 6 hours following the ingestion of 25 g of lactose. Bloating, flatulence, abdominal pain and cramps are assigned using a score of 0 if there were no symptoms, 1 for mild symptoms, 2 for moderate symptoms and 3 for severe symptoms.

To assess the microbiota profile, DNA is extracted from faecal samples using a 96-well PowerSoil DNA Isolation Kit (MO-BIO). A minimum of one sample-well per plate is kept empty to serve as a negative control during PCR. PCR is done with the forward primer S-D-Bact-0341-b-S-17 and reverse primer S-D-Bact-0785-a-A-21 with Illumina adapters attached [11]. These are universal bacterial 16S rDNA primers, which target the V3-V4 region. Following PCR program is used: 98 °C for 30 sec, 25x (98 °C for 10 s, 55 °C for 20 s, 72 °C for 20 s), 72 °C for 5 min. Amplification is verified by running the products on a 1 % agarose gel. Barcodes are added in a nested PCR using the Nextera Index Kit V2 (Illumina) with the following PCR program: 98 °C for 30 sec, 8x (98 °C for 10 s, 55 °C for 20 s, 72 °C for 20 s), 72 °C for 5 min. Attachment of primers is verified by running the products on a 1 % agarose gel. Products from the nested PCR are normalized using the SequalPrep Normalization Plate Kit and pooled. Pooled libraries are concentrated by evaporation and the DNA concentration of pooled libraries is measured on a Qubit fluorometer using the Qubit High Sensitivity Assay Kit (Thermo Fisher Scientific). Sequencing is done on a MiSeq desktop sequencer using the MiSeq Reagent Kit V3 (Illumina) for 2 x 300 bp paired-end sequencing. The 64-bit version of USEARCH is used for bioinformatical analysis of the sequence data.

Bacterial beta-galactosidase is assessed as measures of stool enzyme activity. Faecal beta-galactosidase is measured by adding 20 µl of faeces homogenised in buffer to 480 µg of O-nitrophenyl-beta-D-galactopyranoside in sodium phosphate buffer (pH 7.0). The reaction is allowed to proceed at 45 °C for 10 min. Sodium carbonate (1 M) is added to stop the reaction. Optical density at 420 nm is subsequently read and beta-galactosidase activity is reported as units/g of stool.

The results show that oral ingestion of HMOs reduce bowel movement frequency and improve stool consistency. The HMOs also modulate the intestinal microbiota, and specifically stimulate the growth of bifidobacteria. The increase in abundance of bifidobacteria occurs at the expense of *Escherichia* and *Clostridium,* which are reduced. The level of bifidobacteria correlated positively with beta-galactosidase, and negatively with hydrogen gas production. Additionally, the results show that symptoms of abdominal pain, cramping, bloating, and flatulence normally provoked by lactose consumption are improved after HMO supplementation in individuals with lactose intolerance. Collectively, HMOs are able to increase bifidobacteria and change the intestinal environment, and by this, reduce gastrointestinal symptoms and in particular diarrhoea.

### Example 2 - Nutritional composition

A ready to feed nutritional composition is prepared from water, maltodextrin, corn syrup, sugar, milk protein concentrate, vegetable oil (canola, high oleic sunflower and corn), soy protein isolate, acacia gum, flavours, HMSs/HMOs, potassium citrate, magnesium phosphate, cellulose gel and gum, calcium carbonate, sodium ascorbate, soy lecithin, choline bitartrate, calcium phosphate, alpha-tocopheryl acetate, ascorbic acid, carrageenan gum, ferric pyrophosphate, flavours, sweeteners (Stevia), vitamin A palmitate, niacinamide, vitamin D3, calcium pantothenate, manganese sulphate, copper sulphate, pyridoxine hydrochloride, thiamine hydrochloride, beta carotene, riboflavin, chromium chloride, folic acid, biotin, potassium iodide, phytonadione, sodium selenite, sodium molybdate, vitamin B12.

The composition provides a nutritional supplement which is a good source of protein, low in fat, vitamins, minerals and antioxidants, and meets FODMAP criteria. Further, the composition contains HMSs and/or HMOs which are able to promote the growth of beneficial intestinal bacteria and modulate chronic inflammation.

### Example 3 - Capsule composition

A capsule is prepared by filling about 1 g of HMS/HMO into a 000 gelatine capsule using a filing machine. The capsules are then closed. The HMS/HMO are in free flowing, powder form.

### Example 4 - Mucosal barrier function

2'-FL and LNnT are tested with respect to their ability to induce MUC2, TFF3, EIMβ, CHST5, and GAL3ST2 expression in the human LS174T cell culture model of goblet cells. The human LS174T cell line is obtained from the American Type Culture Collection (ATCC). LS174T cells are maintained in minimum essential medium (MEM) supplemented according to instructions at 37 °C in 5% CO₂. 2'-FL and LNnT are dissolved in cell culture grade water to the required concentration. The LS174T cells are treated with the HMO solution containing 0 or 5 mg HMO/ml.

The LS174T cells are collected and suspended in Trizol reagent and total RNA is isolated using an RNA analysis kit (Qiagen) according to the manufacturer's instructions and the RNA isolates are quantified using Nanodrop analysis (Thermo Fisher Scientific). RNA isolates are reverse transcribed using a high capacity cDNA Reverse Transcription Kit (Applied Biosystems) to create cDNA, which is then used to assess gene expression via quantitative RT-PCR.

For the quantitative RT-PCR, specific TaqMAN gene expression assays are obtained from Applied Biosystems, which include expression assays for MUC2, TFF3, CHST5 and GAL3ST2. Quantitative real-time PCR is performed using TaqMAN PCR Master Mix (Applied Biosystems). Reactions are run in duplicates in a 384-well plate using an Applied Biosystems 7900HT Fast Real-Time PCR System. The results are analysed using SDS 2.3 software and calculated by delta delta Ct method. All samples are normalized to Gus-β expression and fold induction is calculated over untreated controls. Gene expression is expressed as fold increase compared to HMO-free control cells. The experiment is repeated three times.

The results indicate that treatment with 2'-FL and LNnT increases the expression of the MUC2 and TFF3 genes compared to control cultures. Increased expression of goblet cell genes is specific and not universal, as evidenced by the minimal induction or lack of induction of CHST5 and GAL3ST2, respectively. MUC2 and TFF3 are key components of the mucosal barrier and improve mucosal barrier function.

### Refrence example 1- Human trial in IBS-D patients

A total of 60 male and female IBS patients are recruited to participate in the study. After a screening visit and run-in period of 1-2 weeks, the patients are selected. The patients are randomized into three groups, each of 20 patients, with two groups consuming the treatment product and one group the placebo product for 4 weeks. The treatment product contains either 5 or 10 grams of a combination of 2'-FL and LNnT in a 4:1 ratio, while the placebo product contains 5 grams of glucose. Both products are in powder form in a unit dosage container.

The patients are eligible to participate if they are at an age between 18-60 years, fulfil definition of IBS-D, IBS-C or IBS-A/M according to the Rome IV criteria for IBS and have a global IBS-SSS score of >174 during the 2 weeks run-in period. All recruited patients are able and willing to understand and comply with the study procedures. Patients are excluded if: they have any known gastrointestinal disease(s) that may cause symptoms or interfere with the trial outcome, in particular lactose intolerance and coeliac disease; they have participated in a clinical study one month prior to screening visit; they have abnormal results in the screening tests which are clinically relevant for study participation; they are suffering for a severe disease such as malignancy, diabetes, severe coronary disease, kidney disease, neurological disease, or severe psychiatric disease or any condition which can confound the results of the study; used highly dosed probiotic supplements (yoghurt allowed) for 1 months prior to the study; consumed antibiotic drugs 1 months prior to the study; consumed on a regular basis any medication that might interfere with symptom evaluation 2 weeks prior to the study; diagnosed with and treated for IBS for more than 10 years; and pregnant or lactating.

At the screening visit, clinical and medical history and concomitant medication is registered. IBS diagnostic criteria will be assessed and part 2 of the IBS-SSS questionnaire will be completed.

A faecal sample kit is distributed together with the Bristol Stool Form Scale (BSFS) and Bowel Movement Diary (BMD) to be filled in during the 7 days just prior to visit 2. Patients will be asked to register their diet 3 days just prior to visit 2, and will be reminded not to change their usual diet during the study.

At the second visit, eligibility criteria are checked and eligible subjects are randomised to the three arms in the trial. A physical examination is done and a number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Based on clinical symptoms and data from questionnaires, patients are characterised into one of the three following groups; diarrhoea predominant (IBS-D), constipation predominant (IBS-C) or alternating/mixed (IBS-A/M). This enables allocation of patients from each subgroup into the intervention groups. When allocated to the groups patients are provided with either treatment or placebo products. Sigmoidoscopy is performed and mucosal biopsies and faecal aspirates taken. Patients are asked about any adverse events and any changes in their usual medication. The BSFS and BMD is collected and new forms, to be filled in daily during the intervention period, are distributed. Faecal samples are collected and equipment for new samples are distributed. Blood samples are collected for routine clinical chemistry and haematology and biomarker analysis and a saliva sample is collected to analyse FUT2 secretor status. Diet records are collected, and patients are asked to register their diet for 3 days just prior to visit 3. Patients are reminded not to change their usual diet during the study.

At the third visit, a physical examination is performed and a number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Remaining study products and compliance diaries are collected to check compliance. Blood samples are collected for routine clinical chemistry and haematology and biomarker analysis, and sigmoidoscopy is performed and mucosal biopsies and faecal aspirates taken. Patients are asked about any adverse events and any changes in their usual medication. Faecal samples are collected and equipment for collecting new samples distributed. The BSFS and BMD is collected and new forms, to be filled in during the 7 days just prior to visit 4, are distributed. Diet records are collected, and patients are reminded not to change their usual diet during the study.

The treatment period lasts 4 weeks, the patients are administered 5 or 10 g of mix of 2'-FL + LNnT or 5 g of glucose daily. Patients are instructed to consume the products in the morning with breakfast. Compliance is monitored through the interactive internet enabled system.

At the end of the study, each patient has an exit visit with the medical team. Faecal samples and blood samples are collected and analysed as before. A number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Patients are asked about any adverse events and any changes in their usual medication or diet, and the BSFS and BMD is collected.

To assess the microbiota profile, DNA is extracted from faecal samples using a 96-well PowerSoil DNA Isolation Kit (MO-BIO). A minimum of one sample-well per plate is kept empty to serve as a negative control during PCR. PCR is done with the forward primer S-D-Bact-0341-b-S-17 and reverse primer S-D-Bact-0785-a-A-21 with Illumina adapters attached (Klindworth et al. *Nucleic Acids Res.* 41, e1 (2013)). These are universal bacterial 16S rDNA primers, which target the V3-V4 region. Following PCR program is used: 98 °C for 30 sec, 25x (98 °C for 10 s, 55 °C for 20 s, 72 °C for 20 s), 72 °C for 5 min. Amplification is verified by running the products on a 1 % agarose gel. Barcodes are added in a nested PCR using the Nextera Index Kit V2 (Illumina) with the following PCR program: 98 °C for 30 sec, 8x (98 °C for 10 s, 55 °C for 20 s, 72 °C for 20 s), 72 °C for 5 min. Attachment of primers is verified by running the products on a 1 % agarose gel. Products from the nested PCR are normalized using the SequalPrep Normalization Plate Kit and pooled. Pooled libraries are concentrated by evaporation and the DNA concentration of pooled libraries is measured on a Qubit fluorometer using the Qubit High Sensitivity Assay Kit (Thermo Fisher Scientific). Sequencing is done on a MiSeq desktop sequencer using the MiSeq Reagent Kit V3 (Illumina) for 2 x 300 bp paired-end sequencing. The 64-bit version of USEARCH is used for bioinformatical analysis of the sequence data.

The treatment patients report a reduction in pain/visceral sensitivity, a reduction in bowel movement frequency and an improvement in stool consistency as compared to the placebo group. The blood biomarker analysis indicates that the treatment patients have reduced levels of inflammatory markers, and the biopsy analysis reveals reduced gut permeability indicating an improved mucosal barrier, and reduced evidence of mast cell activation and degranulation. The faecal analysis indicates that the treatment patients have reduced levels of intestinal dysbiosis and a higher level of bifidobacteria; especially those of the *Bifidobacterium adolescentis* phylogenetic group initially and subsequently *Bifidobacterium longum* and *Bifidobacterium bifidum.*

### Reference example 2 - Human trial in antibiotic treated children

A total of 40 children of age 5 to 10 years are recruited to participate in the study. The children are commencing a broad spectrum, antibiotic therapy prescribed by a doctor for an infectious disorder. All recruited children and their caretakers are able and willing to understand and comply with the study procedures. Children are excluded if: they have participated in a clinical study one month prior to screening visit; they are suffering from a severe disease such as gastro-intestinal diseases, malignancy, diabetes, severe coronary disease, kidney disease, neurological disease, or severe psychiatric disease or any condition which can confound the results of the study; used highly dosed probiotic supplements (yoghurt allowed) for 3 months prior to the study; consumed antibiotic drugs 3 months prior to the study, and consumed on a regular basis any medication that might interfere with symptom evaluation 2 weeks prior to the study.

At a screening visit, medical history and concomitant medication is registered. Additionally, eligibility criteria are checked and eligible subjects are randomised into two groups, each of 20 children. The treatment period (6 weeks) is divided into two, as follows.
- Period 1 (2 weeks): Group 1 (placebo), Group 2 (treatment product),
- Period 2 (4 weeks): Group 1 (placebo), Group 2 (treatment product).

The treatment product contains 5 grams of a combination of 2'-FL and LNnT (4:1 mass ratio), while the placebo product contains 5 grams of glucose. Both products are in powder form in a sachet. The products are each administered daily as a bolus at breakfast, and diet is not controlled; however, the participants are asked not to change their normal diet over the course of the study.

At the initial visit, faecal sample kits and either treatment or placebo products are distributed. Each child's caretaker is instructed to keep the faecal samples in the freezer until the next visit. The children and caretaker are reminded not to change the children's usual diet during the study. A faecal sample is collected at this visit and stored at -80° C until analysis.

The study runs for two plus four weeks with the children consuming either placebo and/or treatment product daily. Compliance is monitored through the interactive internet enabled system.

The participants also use the system to record:
- Bristol Stool Form Scale (BSFS) information,
- Questionnaire for Paediatric Functional GI Disorders (QPFG).

The QPFG questionnaire covers dimensions such as abdominal pain and discomfort, bowel movement, and other gastrointestinal symptoms.

At the end of period 1, faecal samples are collected and new treatment or placebo products are distributed. At the end of period 2 (exit visit), each children has a visit with the medical team and faecal samples are collected. After 6 months, faecal samples are collected at a follow up visit.

To assess the microbiota profile, DNA is extracted from the faecal samples using a 96-well PowerSoil DNA Isolation Kit (MO-BIO). A minimum of one sample-well per plate is kept empty to serve as a negative control during PCR. PCR is done with the forward primer S-D-Bact-0341-b-S-17 and reverse primer S-D-Bact-0785-a-A-21 (Klindworth et al. *Nucleic Acids Res.* 41, e1 (2013)) with Illumina adapters attached. These are universal bacterial 16S rDNA primers, which target the V3-V4 region. The following PCR program is used: 98 °C for 30 sec, 25x (98° C for 10 s, 55 °C for 20 s, 72 °C for 20 s), 72 °C for 5 min. Amplification is verified by running the products on a 1 % agarose gel. Barcodes are added in a nested PCR using the Nextera Index Kit V2 (Illumina) with the following PCR program: 98 °C for 30 sec, 8x (98 °C for 10 s, 55 °C for 20 s, 72 °C for 20 s), 72 °C for 5 min. Attachment of primers is verified by running the products on a 1 % agarose gel.

Products from the nested PCR are normalized using the SequalPrep Normalization Plate Kit and pooled. Pooled libraries are concentrated by evaporation and the DNA concentration of pooled libraries wisas measured on a Qubit fluorometer using the Qubit High Sensitivity Assay Kit (Thermo Fisher Scientific). Sequencing is done on a MiSeq desktop sequencer using the MiSeq Reagent Kit V3 (Illumina) for 2 x 300 bp paired-end sequencing. The 64-bit version of USEARCH (Edgar, 2013) is used for bioinformatical analysis of the sequence data.

The faecal analyses reveal that HMOs are able to prevent antibiotic mitigated dysbiosis and enhance a favourable microbiota composition by increasing the abundance of bifidobacteria and especially the *Bifidobacterium adolescentis* phytogenic group during and post antibiotic therapy. Further the children have reduced incidence of diarrhoea.

## Claims

1. One or more human milk oligosaccharides (HMOs) for use in the prophylaxis or treatment of non-infectious diarrhoea in a human, wherein the human is a lactose intolerant patient.

2. A synthetic composition for use in the prophylaxis or treatment of non-infectious diarrhoea in a human, wherein the human is a lactose intolerant patient, the composition comprising an effective amount of a human milk oligosaccharide (HMO).

3. One or more HMOs for the use according to claim 1 or the synthetic composition for the use according to claim 2, wherein the human has intestinal dysbiosis and/or an impaired mucosal barrier.

4. One or more HMOs or the synthetic composition for the use according to any one of the preceding claims, wherein the bowel movement frequency is reduced and stool consistency is improved.

5. One or more HMOs for the use according to any of claims 1 and 3 to 4 effective to increase (i) the abundance, particularly the relative abundance, of bifidobacteria, and/or (ii) the beta-galactosidase activity, in the gastrointestinal tract of the human.

6. The synthetic composition for the use according to any one of claims 2 to 4, comprising an amount of HMO effective to increase (i) the abundance, particularly the relative abundance, of bifidobacteria, and/or (ii) the beta-galactosidase activity, in the gastrointestinal tract of the human.

7. One or more HMOs for the use according to claim 5 or the synthetic composition for the use according to claim 6, wherein the bifidobacteria, in the initial treatment period, are those of the phylogenetic *Bifidobacterium adolescentis* group and, after about 14 days of treatment, are *Bifidobacterium longum* and/or *Bifidobacterium bifidum.*

8. One or more HMOs or the synthetic composition for the use according to claim 7, wherein the bifidobacterium of the phylogenetic *Bifidobacterium adolescentis* group is *Bifidobacterium pseudocatenulatum* and/or *Bifidobacterium adolescentis.*

9. One or more HMOs for the use according to any one of claims 1, 3 to 5, 7 and 8, wherein the HMO is a fucosylated or a non-fucosylated neutral HMO.

10. One or more HMOs for the use according to any one of claims 1, 3 to 5, 7 and 8, wherein the HMO is 2'-FL, 3-FL, DFL, LNnT, LNT, 3'-SL, 6'-SL or LNFP-I, or a mixture thereof.

11. One or more HMOs for the use according to claim 10, wherein the one or more HMOs is a mixture comprising or essentially consisting of 2'-FL and/or DFL, and LNnT and/or LNT.

12. The synthetic composition for the use according to any one of the claims 2 to 4 and 6 to 8, wherein the HMO is a fucosylated or a non-fucosylated neutral HMO.

13. The synthetic composition for the use according to any one of claims 2 to 4 and 6 to 8, wherein the HMO is 2'-FL, 3-FL, DFL, LNnT, LNT, 3'-SL, 6'-SL or LNFP-I, or a mixture thereof.

14. The synthetic composition for the use according to claim 13, wherein the HMO is a mixture comprising or essentially consisting of 2'-FL and/or DFL, and LNnT and/or LNT.

## Patentansprüche

1. Ein oder mehrere humane Milcholigosaccharide (HMOs) zur Verwendung in der Prophylaxe oder Behandlung von nicht infektiöser Diarrhö bei einem Menschen, wobei der Mensch ein Patient mit Lactoseintoleranz ist.

2. Eine Synthetische Zusammensetzung zur Verwendung in der Prophylaxe oder Behandlung von nicht infektiöser Diarrhö bei einem Menschen, wobei der Mensch ein Patient mit Lactoseintoleranz ist, wobei die Zusammensetzung eine wirksame Menge eines humanen Milcholigosaccharids (HMO) umfasst.

3. Ein oder mehrere HMOs zur Verwendung nach Anspruch 1 oder die synthetische Zusammensetzung zur Verwendung nach Anspruch 2, wobei der Mensch an intestinaler Dysbiose und/oder einer gestörten Schleimhautbarriere leidet.

4. Ein oder mehrere HMOs oder die synthetische Zusammensetzung zur Verwendung nach einem der vorhergehenden Ansprüche, wobei die Stuhlganghäufigkeit reduziert wird und die Stuhlkonsistenz verbessert wird.

5. Ein oder mehrere HMOs zur Verwendung nach einem der Ansprüche 1 und 3 bis 4, welches oder welche wirksam ist oder sind, um (i) die Abundanz, insbesondere die relative Abundanz, von Bifidobakterien, und/oder (ii) die beta-Galactosidase-Aktivität, im Gastrointestinaltrakt des Menschen zu erhöhen.

6. Die Synthetische Zusammensetzung zur Verwendung nach einem der Ansprüche 2 bis 4, umfassend eine Menge an HMO, die wirksam ist, um (i) die Abundanz, insbesondere die relative Abundanz, von Bifidobakterien, und/oder (ii) die beta-Galactosidase-Aktivität, im Gastrointestinaltrakt des Menschen zu erhöhen.

7. Ein oder mehrere HMOs zur Verwendung nach Anspruch 5 oder die synthetische Zusammensetzung zur Verwendung nach Anspruch 6, wobei die Bifidobakterien, in der anfänglichen Behandlungsperiode, jene der phylogenetischen *Bifidobacterium adolescentis* Gruppe sind, und, nach ungefähr 14 Tagen der Behandlung, *Bifidobacterium longum* und/oder *Bifidobacterium bifidum* sind.

8. Ein oder mehrere HMOs oder die synthetische Zusammensetzung zur Verwendung nach Anspruch 7, wobei das Bifidobacterium der phylogenetischen *Bifidobacterium adolescentis* Gruppe *Bifidobacterium pseudocatenulatum* und/oder *Bifidobacterium adolescentis* ist.

9. Ein oder mehrere HMOs zur Verwendung nach einem der Ansprüche 1, 3 bis 5, 7 und 8, wobei das HMO ein fucosyliertes oder ein nicht fucosyliertes neutrales HMO ist.

10. Ein oder mehrere HMOs zur Verwendung nach einem der Ansprüche 1, 3 bis 5, 7 und 8, wobei das HMO 2'-FL, 3-FL, DFL, LNnT, LNT, 3'-SL, 6'-SL oder LNFP-I, oder eine Mischung davon ist.

11. Ein oder mehrere HMOs zur Verwendung nach Anspruch 10, wobei das eine oder die mehreren HMOs eine Mischung ist oder sind, umfassend oder im Wesentlichen bestehend aus 2'-FL und/oder DFL, und LNnT und/oder LNT.

12. Die Synthetische Zusammensetzung zur Verwendung nach einem der Ansprüche 2 bis 4 und 6 bis 8, wobei das HMO ein fucosyliertes oder ein nicht fucosyliertes neutrales HMO ist.

13. Die Synthetische Zusammensetzung zur Verwendung nach einem der Ansprüche 2 bis 4 und 6 bis 8, wobei das HMO 2'-FL, 3-FL, DFL, LNnT, LNT, 3'-SL, 6'-SL oder LNFP-I, oder eine Mischung davon ist.

14. Die Synthetische Zusammensetzung zur Verwendung nach Anspruch 13, wobei das HMO eine Mischung ist, umfassend oder im Wesentlichen bestehend aus 2'-FL und/oder DFL, und LNnT und/oder LNT.

## Revendications

1. Un ou plusieurs oligosaccharides de lait humain (HMO) pour leur utilisation dans la prophylaxie ou le traitement d'une diarrhée non infectieuse chez un être humain, où l'être humain est un patient intolérant au lactose.

2. Une composition synthétique pour son utilisation dans la prophylaxie ou le traitement d'une diarrhée non infectieuse chez un être humain, où l'être humain est un patient intolérant au lactose, la composition comprenant une quantité efficace d'un oligosaccharide de lait humain (HMO).

3. Un ou plusieurs HMO pour leur utilisation selon la revendication 1 ou la composition synthétique pour son utilisation selon la revendication 2, où l'être humain présente une dysbiose intestinale et/ou une barrière muqueuse altérée.

4. Un ou plusieurs HMO ou composition synthétique pour leur utilisation selon l'une quelconque des revendications précédentes, où la fréquence de défécation est réduite et la consistance des selles est améliorée.

5. Un ou plusieurs HMO pour leur utilisation selon l'une quelconque des revendications 1 et 3 à 4, efficaces pour augmenter (i) l'abondance, notamment l'abondance relative, de bifidobactéries, et/ou (ii) l'activité bêta-galactosidase, dans le tractus gastro-intestinal de l'être humain.

6. La composition synthétique pour son utilisation selon l'une quelconque des revendications 2 à 4, comprenant une quantité de HMO efficace pour augmenter (i) l'abondance, notamment l'abondance relative, de bifidobactéries, et/ou (ii) l'activité bêta-galactosidase, dans le tractus gastro-intestinal de l'être humain.

7. Un ou plusieurs HMO pour leur utilisation selon la revendication 5 ou la composition synthétique pour son utilisation selon la revendication 6, où les bifidobactéries, durant la période de traitement initiale, sont celles du groupe phylogénétique *Bifidobacterium adolescentis* et, après environ 14 jours de traitement, sont celles de *Bifidobacterium longum* et/ou *Bifidobacterium bifidum.*

8. Un ou plusieurs HMO ou la composition synthétique pour leur utilisation selon la revendication 7, où la bifidobactérie du groupe phylogénétique *Bifidobacterium adolescentis* est *Bifidobacterium pseudocatenulatum* et/ou *Bifidobacterium adolescentis.*

9. Un ou plusieurs HMO pour leur utilisation selon l'une quelconque des revendications 1, 3 à 5, 7 et 8, où le HMO est un HMO fucosylé ou non fucosylé neutre.

10. Un ou plusieurs HMO pour leur utilisation selon l'une quelconque des revendications 1, 3 à 5, 7 et 8, où le HMO est un 2'-FL, 3-FL, DFL, LNnT, LNT, 3'-SL, 6'-SL ou LNFP-I, ou un mélange de ceux-ci.

11. Un ou plusieurs HMO pour leur utilisation selon la revendication 10, où les un ou plusieurs HMO sont un mélange comprenant ou consistant essentiellement de 2'-FL et/ou DFL, et de LNnT et/ou LNT.

12. La composition synthétique pour son utilisation selon l'une quelconque des revendications 2 à 4 et 6 à 8, où le HMO est un HMO fucosylé ou non fucosylé neutre.

13. La composition synthétique pour son utilisation selon l'une quelconque des revendications 2 à 4 et 6 à 8, où le HMO est un 2'-FL, 3-FL, DFL, LNnT, LNT, 3'-SL, 6'-SL ou LNFP-I, ou un mélange de ceux-ci.

14. La composition synthétique pour son utilisation selon la revendication 13, où le HMO est un mélange comprenant ou consistant essentiellement de 2'-FL et/ou DFL, et de LNnT et/ou LNT.
